# EUROPEAN PATENT SPECIFICATION

(11) **EP 3 069 750 B1**
(45) Date of publication and mention of the grant of the patent: **31.01.2018**
(21) Application number: 16159713.3
(22) Date of filing: 10.03.2016
(51) Int. Cl.: A61M 25/09

(54) **FLEXIBLE HYBRID WIRE GUIDE**
FLEXIBLE HYBRIDE DRAHTFÜHRUNG
GUIDE DE FIL HYBRIDE FLEXIBLE

(30) Priority: 12.03.2015 US 201562131923 P
(43) Date of publication of application: 21.09.2016
(73) Proprietor: Cook Medical Technologies LLC, Bloomington, IN 47404 (US)
(72) Inventor: IRWIN, Nathaniel, A, Bloomington, IN Indiana 47401 (US); ELSESSER, James, C, Bloomington, IN Indiana 47401 (US); CAGE, Logan, Bloomington, IN Indiana 47401 (US); KAY Jr., Thomas, A, Bloomington, IN Indiana 47401 (US); MERK, James, C, Terre Haute, IN Indiana 47802 (US)
(74) Representative: Maitland, Matthew

(56) References cited:
- EP-A1- 1 388 348
- EP-A1- 1 839 697
- EP-A1- 2 140 904
- US-A- 5 437 288
- US-A1- 2007 135 732

## Description

### Technical Field

The present disclosure relates generally to wire guides for medical applications, and more particularly to a hybrid wire guide that takes advantage of the pushability of stainless steel and the flexibility of nitinol.

### Background

In the diagnosis and intervention of peripheral arterial disease, wire guides are commonly used for access, navigation, and treatment. In peripheral cases, the wire guide is expected to traverse a long, tortuous pathway while being durable. In recent years, hybrid wires have been introduced to take advantage of different material properties to accomplish these tasks. For example, stainless steel/nitinol wires have been used to take advantage of the pushability of stainless steel and the flexibility of nitinol. One such example is described in U.S. Patent 8,348,860. One of the challenges for joining these two materials is having a smooth flexible transition between the joined stainless steel and nitinol wires. If the flexibility of the two sections are not transitioned well, the result can be imagined as being similar to a stiff fishing pole with a flexible line extending from one end, where the stiff fishing pole represents a proximal section of stainless steel and the flexible line represents a distal section of nitinol. In a clinical setting, having a poor transition may prevent a wire from successfully advancing around tight corners/bends in a patient's anatomy.

Conventional wire guides often include a core member that is substantially made from a single material. In particular, to enhance the pushability of the guide wire, a material having a relatively high elastic modulus (stainless steel) is used as the material of the core member. The wire guide including such a core member may, however, result in a distal end portion with lower than desirable flexibility. On the otherhand, if a material having a relatively low elastic modulus (nitinol) is used as the material of the core member for increasing the flexibility of the distal end portion of the wire guide, the pushability of the proximal end portion of the wire guide may be degraded. In this way, it has been regarded as difficult to satisfy both requirements associated with flexibility and pushability by using a core member made from a single material.

The present disclosure is directed toward one or more problems set forth above.

A variety of guide wire constructions are known from the prior art. For example, EP 1 388 348 A1 discloses a guide wire that includes a first wire, disposed on the distal side, and a second wire, disposed on the proximal side and made from a material having an elastic modulus larger than that of the first wire. The first wire and the second wire are joined to each other by welding. The second wire has, in the vicinity of the welded portion, a small cross-sectional area portion having a cross-sectional area smaller than that of a proximal end portion of the first wire. The outer diameter of the small cross-sectional area portion is gradually reduced in the direction toward the distal end. The first wire may be made from a superelastic alloy, whereas the second wire may be made from a stainless steel. The first wire and the second wire may be welded to each other by a butt resistance welding process. The document asserts that, since the change in rigidity of the guide wire becomes smooth in the longitudinal direction, the operationality and kink resistance of the guide wire are improved.

Reference is further directed to US 5,437,288, which discloses a catheter guidewire that includes an elongate non-coiled wire having a proximal portion, a distal tip and a flexible portion located between the proximal portion and the distal tip having axially spaced grooves cut therein. The method for making the guidewire includes burnishing one end of a wire to create a rounded distal tip and cutting a predetermined pattern of axially spaced grooves in a length of wire adjacent to the distal tip to create a flexible portion.

Reference is additionally directed to US 2007/135732 A1, which discloses a support member for providing core support to an elongated medical device, such as a catheter, wire guide, and the like. The support member includes a generally cylindrical substrate having a proximal end and a distal end, and having a mass such that the respective proximal and distal ends are visible under medical imagery. The substrate is sized to be received in a lumen of the medical device, and has a plurality of grooves, axially disposed along the length of the substrate. The support member may have a greater number of grooves per unit length of the substrate at the distal end than at the proximal end, thereby imparting an increased flexibility to the distal end when compared to the proximal end.

### Summary

A wire guide includes a proximal end of a nitinol wire joined to a distal end of a stainless steel wire at a weld. The stainless steel wire includes a flex transition segment that terminates at the weld, and defines a plurality of flexibility enhancing voids distributed in a pattern along a centerline so that a flexibility of the flex transition segment changes toward a match of a flexibility of the nitinol wire over a tracking segment that extends distally from the weld. The respective shapes of the flexibility enhancing voids change responsive to flexure of the flex transition segment away from a straight configuration.

In some examples, the flexibility of the flex transition segment may, for example, gradually increase with decreasing distance to the weld. The increase in flexibility may be strictly increasing, for instance flexibility may increase monotonically. In some cases, the increase in flexibility may be generally linear.

In some examples, the spacing of the flexibility enhancing voids may decrease with decreasing distance to the weld. In addition, or instead, the depth of the flexibility enhancing voids may increase with decreasing distance to the weld.

In some examples, there may be multiple flexibility enhancing voids at each of a number of longitudinal locations distributed over the flex transition segment. The number of flexibility enhancing voids at each such location may increase with decreasing distance to the weld. In addition, or instead, the spacing between such locations may decrease with decreasing distance to the weld. In addition, or instead, the depth of the flexibility enhancing voids at each such location may increase with decreasing distance to the weld.

In some examples, the flexibility enhancing voids may, for example, be formed in a substantially cylindrical exterior surface of the flex transition segment (with some allowance being given for a small amount of tapering of this "substantially cylindrical" exterior surface). In some examples, the flex transition segment may have an exterior surface that includes a plurality of substantially cylindrical surfaces and the flexibility enhancing voids may be formed in this plurality of substantially cylindrical surfaces.

In some examples, most, or substantially all of the flexibility enhancing voids may have an extent in the longitudinal direction of the wire guide that is smaller than the radius of the flex transition segment. Additionally, or instead, each of the flexibility enhancing voids may extend only part-way around the circumference of the flex transition segment, for instance each flexibility enhancing void may extend around less than a half of the circumference of the flex transition segment.

In some examples, the flexibility enhancing voids may be configured so as to provide different flexibility in different azimuthal directions. For instance, the flexibility enhancing voids may be configured so as to provide greater flexibility in a particular azimuthal direction and, in some cases, in the opposition azimuthal direction.

### Brief Description of the Drawings

Fig. 1 is a graph of flexibility verses distance along the centerline for a hybrid wire guide according to the prior art and the present disclosure;
Fig. 2 is a schematic view of a wire guide according to the present disclosure as per the graph of Fig. 1;
Fig. 3 is a side schematic view of a wire guide according to the present disclosure;
Fig. 4 is a side schematic view of a wire guide according to another variation of the present disclosure;
Fig. 5 is a side schematic view of a wire guide according to still another variation of the present disclosure;
Fig. 6 is a partial side view of a wire guide according to the present disclosure in a curved configuration;
Fig. 7 is a partial side schematic view of a wire guide according to the present disclosure;
Fig. 8 is a partial side schematic view of a wire guide according to another aspect of the present disclosure;
Fig. 9 is a partial side schematic view of a wire guide according to another aspect of the present disclosure;
Fig. 10 is a partial side schematic view of a wire guide according to still another aspect of the present disclosure;
Fig. 11 is a sectioned view of the wire guide of Fig. 6 as viewed along section line A-A; and
Fig. 12 is a sectioned view through the wire guide of Fig. 6 as viewed along section line B-B.

### Detailed Description

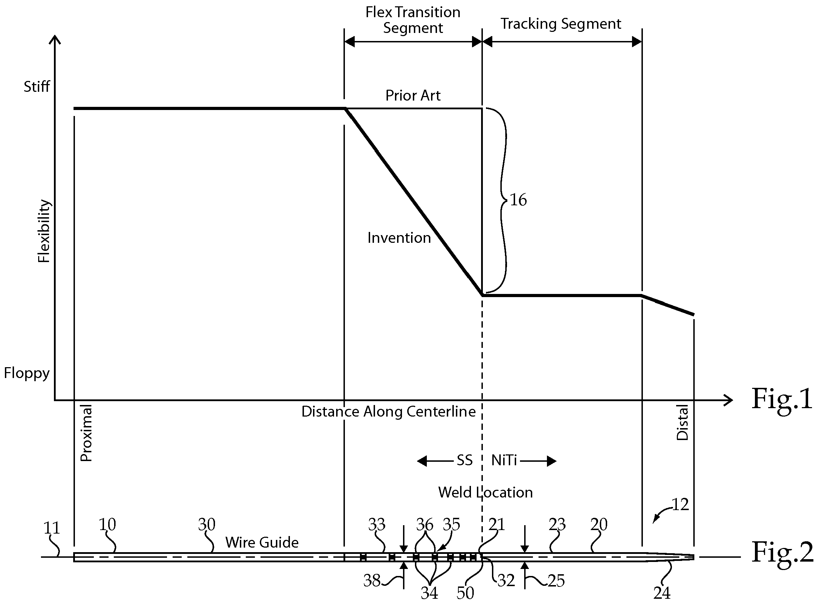

Referring initially to Figs. 1 and 2, an example wire guide 10 according to the present disclosure shows a proximal end 21 of a nitinol wire 20 joined to a distal end 32 of a stainless steel wire 30 at a weld 50. The stainless steel wire 30 includes a flex transition segment 33 that terminates at the weld 50, and defines a plurality of flexibility enhancing voids 34 distributed in a pattern 35 along a centerline 11. The pattern 35 of flexibility enhancing voids 34 causes the flexibility of the flex transition segment 33 to change toward a match of the flexibility of the nitinol wire 20 over a tracking segment 23 that extends distally from the weld 50. The flexibility profile of wire guide 10 is graphed against a distance along a centerline 11 in Fig. 1, with a comparison to an equivalent prior art wire guide that lacks the flexibility enhancing voids 34 of the present disclosure. As shown in Fig. 1, the prior art wire guide results in a large discontinuity 16 in the flexibility of the wire guide on opposite sides of the weld location. The wire guide 10 according to the present disclosure, on the otherhand, relies upon the flexibility enhancing voids 34 to produce a change in flexibility over the flex transition segment 33 that approaches a match of the flexibility of the nitinol wire 20 over tracking segment 23. The respective shapes of the flexibility enhancing voids 34 will change responsive to flexure of the flex transition segment 33 away from a straight configuration 12. Although Figure 1 shows the flexibility of the flex transition segment 33 actually matching the flexibility of the tracking segment 23 at distal end 32, those skilled in the art will appreciate that some discontinuity in the flexibility on opposite sides of the weld 50 would also fall within the scope of the present disclosure. Preferably, any such discontinuity would be small enough to essentially go unnoticed by a trained user of wire guides.

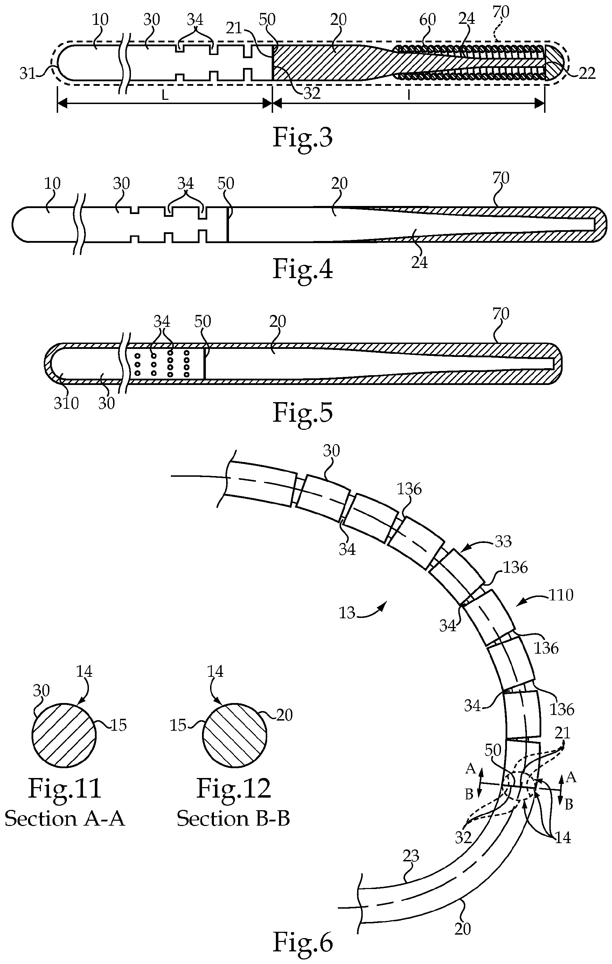

In an effort to generate a weld 50 with potentially less brittle intermetallic material at weld 50, the present disclosure teaches joining of the nitinol wire 20 to the stainless steel wire 30 by a friction weld. Although techniques in accomplishing friction welds need not be taught here, the wires 20 and 30 may be joined by a typical strategy that involves rotating one of the wires about their common centerline 11 as the end of the other wire bears against the exposed end of the rotating wire. The friction between the rotating and non-rotating surfaces creates heat that eventually results in melting of the metals at their interface to produce weld 50. Thus, in the event of a friction weld, the proximal end 21 of the nitinol wire 20 and the distal end 32 of the stainless steel wire 30 may have complementary shapes 14 that are surfaces of rotation about centerline 11. As best shown in Figs. 6, 11 and 12, the distal end 32 of the stainless steel wire 30 and the proximal end 21 of the nitinol wire 20 may be equally sized circles 15 oriented perpendicular to centerline 11 so that the weld 50 lies in a plain perpendicular to centerline 11. Alternatively, as shown by dashed lines in Fig. 6, the complimentary shapes 14 may take the form of complimentary concave and convex shapes as shown by the dotted lines in Fig. 6. The respective proximal end 21 of nitinol wire 20 and distal end 32 of stainless steel wire 30 may include any suitable complimentary shapes 14 to facilitate a friction weld. Nevertheless, other types of welds known in the art for joining the ends of dissimilar metallic wires (e.g. stainless steel and nitinol) would also fall within the intended scope of the present disclosure.

In addition to providing a flex transition segment 33, it may be important for the overall profile of the wire guide 10 in general, and in particular the segments 23, 33 on opposite sides of weld 50, to be free of sharp corners or edges that could inhibit smooth sliding of wire guide 10 through the passageways of a patients body and/or introducers and sheaths and/or undermine passage of a catheter over wire guide 10. This feature may be accomplished by making tracking segment 23 of nitinol wire 20 have a uniform diameter 25 over an entire length of the tracking segment 23. In addition, the flex transition segment 33 may have a maximum diameter 38 that is equal to the uniform diameter 25 of tracking segment 23.

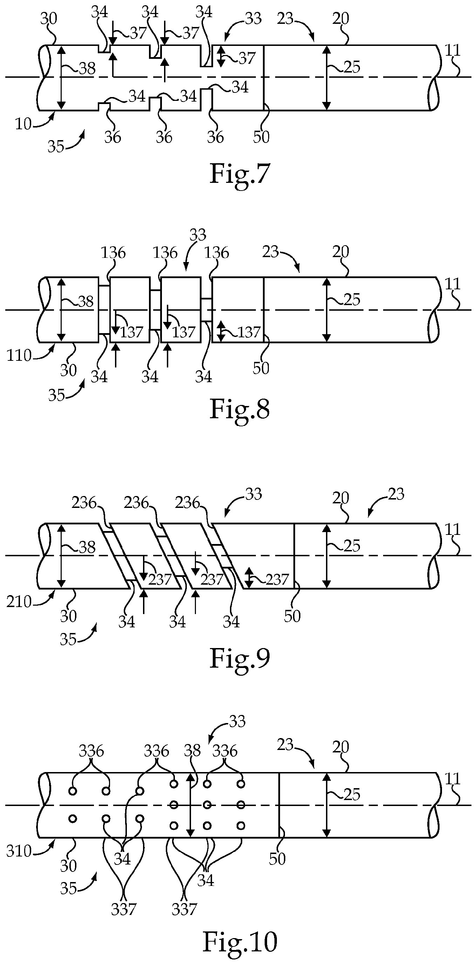

Referring now to Figs. 7-10, several examples of different flexibility enhancing voids 34 according to the present disclosure are illustrated. In Fig. 7, the flex transition segment 33 of the stainless steel wire 30 includes flexibility enhancing voids 34 in the form of a plurality of notches 36. The notches 36 may be oriented perpendicular to centerline 11, or may have some other orientation (e.g., slanted or helical) without departing from the scope of the present disclosure. In order to make the flexibility of the flex transition segment change in the direction of weld 50, a depth 37 of the notches 36 may increase as a distance to the weld 50 decreases. Alternatively, a density of the number of notches 36 per unit length of the flex transition segment 33 may increase in density with a decrease in distance to weld 50. Or, the pattern 35 may exploit changes in both depth 37 and a number of notches 36 per unit length in order to provide a desired change in flexibility over the flex transition segment 33 as best shown in the graph of Fig. 1. Fig. 8 shows an alternative wire guide 110 in which the flexibility enhancing voids 34 take the form of circumferential grooves 136 that are distributed in a pattern to produce a change of flexibility that changes towards a match to the flexibility of the nitinol wire 20 over the tracking segment 23. The circumferential grooves 136 may be oriented perpendicular to centerline 11, or may be angled with regard to centerline 11, and the different grooves could have different angles with regard to centerline 11 without departing from the scope of the present disclosure. The change in flexibility may be accomplished by a depth 137 of the circumferential grooves 136 increasing as the distance to the weld 50 decreases. Alternatively, or in addition, a density of circumferential grooves per unit length may increase as the distance to the weld 50 decreases in order to produce the flexibility of the profile desired in flex transition segment 33. Fig. 9 shows still another wire guide 210 with flexibility enhancing voids 34 taking the form of a helical groove(s) 236. In Fig. 9, the change in flexibility may be accomplished by making a depth 237 of helical groove 236 increase as the distance to the weld 50 decreases. Alternatively, or in addition, the helix angle may be altered (for example to cause the spacing between successive turns of the helix to decrease as the distance to the weld decreases) in addition to or in alternative to the depth 237 in order to achieve the change in flexibility over the flex transition segment 33. For purposes of the present disclosure and the case of a helical groove, each complete rotation about centerline 11 would be considered a separate flexibility enhancing void 34 so that a helical groove that may encircle centerline 11 more than once would be considered to have a plurality of flexibility enhancing voids 34 according to the present disclosure. The respective widths of the notches 36 (Fig. 7), circumferential grooves (Fig. 8) and helical groove 236 (Fig. 9) may also be varied in order to produce a desired change in flexibility and flex transition segment 33 without departing from the present disclosure. Finally, Fig. 10 shows still another wire guide 310 in which the flexibility enhancing voids 34 take the form of holes 336. For purpose of the present disclosure, dimples would be considered holes. In order to provide the change in flexibility in transition segment 33, a depth of the holes may change in the direction of weld 50 and/or, a density in the number of holes 336 per unit length may increase as a distance from weld 50 decreases. In the illustrated embodiment, wire guide 310 includes a plurality of rings of holes 337 in which the number of holes in each ring 337 increases with a decrease in distance to weld 50. The holes 336 may be oriented perpendicular to centerline 11 and may or may not have centerlines that would intersect centerline 11. The present disclosure also contemplates that the size or diameter of the holes 337 may change along centerline 11 in order to provide the change in flexibility over flex transition segment 33. Those skilled in the art will appreciate that wire guides according to the present disclosure may also include combinations of notches, circumferential grooves, spiral grooves, holes and any other shaped removal of material in the flex transition segment 33 in an appropriate pattern 35 to produce the change in flexibility toward a match of the flexibility of the nitinol wire 20 in the region of tracking segment 23.

Although not necessary, the nitinol wire 20 may include a distal segment with a narrowing taper 24 toward a distal end 22 in order to provide an extremely flexible floppy tip at the distal end of wire guide 10. As shown in Fig. 3, wire guide 10 may also include a coil 60. Coil 60 may surround only the distal segment of the nitinol wire 20 that includes narrowing taper 24, or may surround substantially all of nitinol wire 20, or may surround all or a portion of stainless steel wire 30 without departing from the present disclosure. In addition, Fig. 3 is of interest for showing that all or a portion of wire guide 10 may be partially or fully enclosed by a polymer layer 70 that may further be exploited to enhance the ability of the wire guide 10 to slide through a patient's anatomy and/or have a catheter easily slid over the same. Furthermore, a polymer layer 70 may be exploited to smooth out potential surface discontinuities that may result from the flexibility enhancing voids 34 and/or any surface discontinuity at weld 50. Fig. 3 is also of interest for showing that the length L of the stainless steel wire 30 may be the same, shorter or longer than a length 1 of the nitinol wire 20 without departing from the present disclosure. The length L may be measured from a proximal end 31 to a distal end 32 of stainless steel wire 30. Likewise, the length 1 of the nitinol wire 20 may be measured from its proximal end 21 to its distal end 22. Fig. 4 is of interest for showing that a wire guide 10 according to the present disclosure may include a polymer layer 70 that covers only the narrowing taper 24 and distal end segment of the nitinol wire 20. Alternatively, as shown in Fig. 5, a polymer layer 70 may enclose the entire wire guide 310 without departing from the present disclosure.

As best shown in Fig. 6, wire guide 110 is shown in a curved configuration 13 in order to show that the respective shapes of the flexibility enhancing voids 34 change responsive to flexure of the flex transition segment 33 away from a straight configuration 12 as shown in Figure 2. Thus, as shown in Fig. 6, circumferential grooves 136 may have a uniform width around centerline 11 when the flex transition segment 33 is in a straight configuration 12, but may appear to have a more trapezoidal profile as shown in Fig. 6 when in a curved configuration 13. Likewise, one could expect notches 136 on opposite sides of centerline 11 to increase or decrease in width depending upon the direction of curvature of the wire guide 10. In Fig. 10, the holes 336 change in shape from being circular the alternative embodiment wire guide 310, to more elliptical depending upon which direction the flex transition segment 33 of the wire guide is curved. The notches 36 and/or grooves 136,236 may be machined into the stainless steel wire 30 using a known lathe set to a specific CNC program, or in any other manner known in the art. The holes 336, which includes dimples, may be machined into stainless steel wire 30 by micro ball end milling or any other suitable technique known in the art. Alternatively, the holes 336 may be micro machined using a drill press or be cut using a laser cutting machine without departing from the scope of the present disclosure. In any event, the reduction of material in the flex transition segment 33 of the stainless steel wire 30 and the pattern 35 of that reduction material will allow for a more flexible flex transition segment 33 and allow for a better transition to the nitinol portion of the wire guide 10, which will translate to a wire guide 10 with the ability to better navigate around bends in a patient's anatomy.

### Industrial Applicability

The present disclosure finds potential applicability in any wire guide where there is a desire to exploit the desirable characteristics of stainless steel in a proximal portion of a wire guide while retaining the advantages of nitinol in a distal portion of the wire guide, and do so while facilitating a transition in flexibility from the stainless steel to the nitinol that avoids a gross discontinuity in flexibility that would otherwise occur at the weld between the two wires. The present disclosure finds specific applicability to wire guides used in the diagnoses and intervention of peripheral arterial disease where the wire guides are used to access and navigate long tortuous passageways while remaining durable.

For the sake of clarity, a plurality of flexibility enhancing voids 34 according to the present disclosure means something other than the single narrowing of the stainless steel wire as disclosed in U.S. Patent 8,348,860. Also, all wire guides according to the present disclosure include the stainless steel wire 30 being directly welded to the nitinol wire 20 rather than including an intermediate segment of a third metallic material between the stainless steel and nitinol as taught in U.S. Patent application 2014/0246407.

## Claims

1. A wire guide (10) comprising:
a nitinol wire (20);
a stainless steel wire (30);
a proximal end (21) of the nitinol wire (20) being joined to a distal end (32) of the stainless steel wire (30) at a weld (50);
the stainless steel wire (30) includes a flex transition segment (33) that terminates at the weld (50) and defines a plurality of flexibility enhancing voids (34) distributed in a pattern along a centerline (11) so that a flexibility of the flex transition segment (33) changes toward a match of a flexibility of the nitinol wire (20) over a tracking segment (23) that extends distally from the weld (50); and
respective shapes of the flexibility enhancing voids (34) change responsive to flexure of the flex transition segment (33) away from a straight configuration.

2. The wire guide of claim 1, wherein the proximal end of the nitinol wire and a distal end of the stainless steel wire have complimentary shapes that are each surfaces of rotation about the centerline.

3. The wire guide of claim 1 or claim 2, wherein the proximal end of the nitinol wire and the distal end of the stainless steel wire are equally sized circles oriented perpendicular to the centerline.

4. The wire guide of any preceding claim wherein the tracking segment has a uniform diameter over the entire length of the tracking segment;
optionally wherein the flex transition segment has a maximum diameter about equal to the uniform diameter of the tracking segment.

5. The wire guide of any preceding claim, wherein the spacing of the flexibility enhancing voids decreases with decreasing distance to the weld.

6. The wire guide of any preceding claim, wherein the depth of the flexibility enhancing voids increases with decreasing distance to the weld.

7. The wire guide of any preceding claim wherein the flexibility enhancing voids include a plurality of notches;
optionally wherein the notches are oriented perpendicular to the centerline; and/or
wherein a depth of the notches increases as the distance to the weld decreases.

8. The wire guide of any preceding claim wherein the flexibility enhancing voids include a plurality of circumferential grooves;
optionally wherein the circumferential grooves are oriented perpendicular to the centerline; and/or
wherein a depth of the circumferential grooves increases as a distance to the weld decreases.

9. The wire guide of any preceding claim wherein the flexibility enhancing voids include a helical groove with respect to the centerline;
optionally wherein a depth of the helical groove increases as a distance to the weld decreases.

10. The wire guide of any preceding claim wherein the flexibility enhancing voids includes a plurality of holes.

11. The wire guide of claim 10 wherein the plurality of holes includes a plurality of rings of holes; and
each of the rings of holes includes a plurality of holes.

12. The wire guide of claim 11 wherein the number of holes in one of the rings is less than the number of holes in another ring that is closer to the weld.

13. The wire guide of any preceding claim wherein the nitinol wire includes a distal segment with a narrowing taper toward a distal end of the nitinol wire.

14. The wire guide of any preceding claim including a coil that receives, and is attached to, the distal segment of the nitinol wire.

15. The wire guide of any preceding claim including a polymer layer covering at least a portion of the nitinol wire.

## Patentansprüche

1. Drahtführung (10), umfassend:
einen Nitinol-Draht (20);
einen Edelstahldraht (30);
wobei ein proximales Ende (21) des Nitinol-Drahts (20) mit einem distalen Ende (32) des Edelstahldrahts (30) an einer Schweißstelle (50) verbunden ist;
der Edelstahldraht (30) ein Beugungsübergangssegment (33) einschließt, das an der Schweißstelle (50) endet und eine Vielzahl von flexibilitätserhöhenden Lücken (34) definiert, die in einem Muster entlang einer Mittellinie (11) verteilt sind, so dass eine Flexibilität des Beugungsübergangssegments (33) sich zu einer Angleichung an eine Flexibilität des Nitinol-Drahts (20) über ein Nachlaufsegment (23) ändert, das sich distal von der Schweißstelle (50) erstreckt; und
jeweilige Formen der flexibilitätserhöhenden Lücken (34) sich in Reaktion auf die Biegung des Beugungsübergangssegments (33) von einer geraden Konfiguration weg verändern.

2. Drahtführung nach Anspruch 1, wobei das proximale Ende des Nitinol-Drahts und ein distales Ende des Edelstahldrahts komplementäre Formen aufweisen, die jeweils Rotationsflächen um die Mittellinie sind.

3. Drahtführung nach Anspruch 1 oder Anspruch 2, wobei das proximale Ende des Nitinol-Drahts und das distale Ende des Edelstahldrahts gleich bemessene Kreise sind, die senkrecht zu der Mittelllinie orientiert sind.

4. Drahtführung nach einem der vorhergehenden Ansprüche, wobei das Nachlaufsegment einen einheitlichen Durchmesser über die gesamte Länge des Nachlaufsegments aufweist;
wobei gegebenenfalls das Beugungsübergangssegment einen Maximaldurchmesser aufweist, der ungefähr gleich dem einheitlichen Durchmesser des Nachlaufsegments ist.

5. Drahtführung nach einem der vorhergehenden Ansprüche, wobei der Raum zwischen den flexibilitätserhöhenden Lücken mit abnehmender Abstand zu der Schweißstelle abnimmt.

6. Drahtführung nach einem der vorhergehenden Ansprüche, wobei die Tiefe der flexibilitätserhöhenden Lücken mit abnehmendem Abstand zu der Schweißstelle zunimmt.

7. Drahtführung nach einem der vorhergehenden Ansprüche, wobei die flexibilitätserhöhenden Lücken eine Vielzahl von Kerben einschließen;
wobei die Kerben gegebenenfalls senkrecht zu der Mittellinie orientiert sind; und/oder
wobei eine Tiefe der Kerben zunimmt, wenn der Abstand zu der Schweißstelle abnimmt.

8. Drahtführung nach einem der vorhergehenden Ansprüche, wobei die flexibilitätserhöhenden Lücken eine Vielzahl von umlaufenden Rillen einschließen;
wobei die umlaufenden Rillen gegebenenfalls senkrecht zu der Mittellinie orientiert sind; und/oder
wobei eine Tiefe der umlaufenden Rillen zunimmt, wenn ein Abstand zu der Schweißstelle abnimmt.

9. Drahtführung nach einem der vorhergehenden Ansprüche, wobei die flexibilitätserhöhenden Lücken eine spiralförmige Rille in Bezug auf die Mittellinie einschließen;
wobei gegebenenfalls eine Tiefe der spiralförmigen Rille zunimmt, wenn ein Abstand zu der Schweißstelle abnimmt.

10. Drahtführung nach einem der vorhergehenden Ansprüche, wobei die flexibilitätserhöhenden Lücken eine Vielzahl von Löchern einschließen.

11. Drahtführung nach Anspruch 10, wobei die Vielzahl von Löchern eine Vielzahl von Ringen von Löchern einschließt; und
jeder der Ringe von Löchern eine Vielzahl von Löchern einschließt.

12. Drahtführung nach Anspruch 11, wobei die Anzahl der Löcher in einem der Ringe kleiner als die Anzahl der Löcher in einem anderen Ring ist, der sich näher an der Schweißstelle befindet.

13. Drahtführung nach einem der vorhergehenden Ansprüche, wobei der Nitinol-Draht ein distales Segment mit einer enger werden Verjüngung in Richtung eines distalen Endes des Nitinol-Drahts einschließt.

14. Drahtführung nach einem der vorhergehenden Ansprüche, die eine Spule einschließt, die das distale Segment des Nitinol-Drahts aufnimmt und daran befestigt ist.

15. Drahtführung nach einem der vorhergehenden Ansprüche, die eine Polymerschicht einschließt, die mindestens einen Abschnitt des Nitinol-Drahts bedeckt.

## Revendications

1. Fil-guide (10) comprenant :
un fil de Nitinol (20) ;
un fil d'acier inoxydable (30) ;
une extrémité proximale (21) du fil de Nitinol (20) étant assemblée à une extrémité distale (32) du fil d'acier inoxydable (30) au niveau d'une soudure (50) ;
le fil d'acier inoxydable (30) comporte un segment de transition flexible (33) qui se termine à la soudure (50) et définit une pluralité de vides améliorant la flexibilité (34) distribués en un motif le long d'un axe (11) de telle sorte qu'une flexibilité du segment de transition flexible (33) varie jusqu'à correspondre à une flexibilité du fil de Nitinol (20) sur un segment de cheminement (23) qui s'étend distalement depuis la soudure (50) ; et
des formes respectives des vides améliorant la flexibilité (34) changent en réponse à une flexion du segment de transition flexible (33) par rapport à une configuration rectiligne.

2. Fil-guide de la revendication 1, dans lequel l'extrémité proximale du fil de Nitinol et une extrémité distale du fil d'acier inoxydable ont des formes complémentaires qui sont chacune des surfaces de rotation autour de l'axe.

3. Fil-guide de la revendication 1 ou la revendication 2, dans lequel l'extrémité proximale du fil de Nitinol et l'extrémité distale du fil d'acier inoxydable sont des cercles de taille égale orientés perpendiculairement à l'axe.

4. Fil-guide d'une quelconque revendication précédente dans lequel le segment de cheminement a un diamètre uniforme sur la longueur entière du segment de cheminement ;
éventuellement dans lequel le segment de transition flexible a un diamètre maximal approximativement égal au diamètre uniforme du segment de cheminement.

5. Fil-guide d'une quelconque revendication précédente, dans lequel l'espacement des vides améliorant la flexibilité diminue avec une distance décroissante jusqu'à la soudure.

6. Fil-guide d'une quelconque revendication précédente, dans lequel la profondeur des vides améliorant la flexibilité augmente avec une distance décroissante jusqu'à la soudure.

7. Fil-guide d'une quelconque revendication précédente dans lequel les vides améliorant la flexibilité comportent une pluralité d'entailles ;
éventuellement dans lequel les entailles sont orientées perpendiculairement à l'axe ; et/ou
dans lequel une profondeur des entailles augmente lorsque la distance jusqu'à la soudure diminue.

8. Fil-guide d'une quelconque revendication précédente dans lequel les vides améliorant la flexibilité comportent une pluralité de rainures circonférentielles ;
éventuellement dans lequel les rainures circonférentielles sont orientées perpendiculairement à l'axe ; et/ou
dans lequel une profondeur des rainures circonférentielles augmente lorsqu'une distance jusqu'à la soudure diminue.

9. Fil-guide d'une quelconque revendication précédente dans lequel les vides améliorant la flexibilité comportent une rainure hélicoïdale par rapport à l'axe ;
éventuellement dans lequel une profondeur de la rainure hélicoïdale augmente lorsqu'une distance jusqu'à la soudure diminue.

10. Fil-guide d'une quelconque revendication précédente dans lequel les vides améliorant la flexibilité comportent une pluralité de trous.

11. Fil-guide de la revendication 10 dans lequel la pluralité de trous comporte une pluralité d'anneaux de trous ; et
chacun des anneaux de trous comporte une pluralité de trous.

12. Fil-guide de la revendication 11 dans lequel le nombre de trous dans un des anneaux est inférieur au nombre de trous dans un autre anneau qui est plus proche de la soudure.

13. Fil-guide d'une quelconque revendication précédente dans lequel le fil de Nitinol comporte un segment distal avec un cône se rétrécissant vers une extrémité distale du fil de Nitinol.

14. Fil-guide d'une quelconque revendication précédente comportant une spirale qui reçoit le, et est attachée au, segment distal du fil de Nitinol.

15. Fil-guide d'une quelconque revendication précédente comportant une couche de polymère recouvrant au moins une partie du fil de Nitinol.
